# EUROPEAN PATENT APPLICATION

(11) **EP 3 567 602 A1**
(43) Date of publication of application: **13.11.2019**
(21) Application number: 18171846.1
(22) Date of filing: 11.05.2018
(51) Int. Cl.: G16H 50/20

(54) **METHOD FOR UTILISING DATA PROCESSING TECHNOLOGY IN DECISION SUPPORT OF ABNORMAL STATE OF OBJECT**

(71) Applicant: Avaintec Oy, 00180 Helsinki (FI)
(72) Inventor: KULVIK, Martti, 02200 Espoo (FI); LEINONEN, Henrik, 00200 Helsinki (FI); MEZEI, Jozsef, 00530 Helsinki (FI)
(74) Representative: Salomäki, Juha Kari Ensio

(57) **Abstract**

The object of the invention is a method for utilizing data processing technology in decision support of abnormal state of object, which method comprises a computer-based system, knowledge base, data transfer means and an application with a user interface. In the method the application is used to utilize artificial intelligence methods and algorithms providing real-time decision support guidance to the user/users while communicating interactively with the user/users through the user interface.

## Description

### FIELD OF THE INVENTION

The object of the invention is a method as defined in the preamble of claim 1 for utilizing data processing technology in decision support of abnormal state of object. Invention also presents a computer- based arrangement for creating decision support instructions of abnormal state of object. One useful embodiment of the object is a human body where for example the method can be used effectively in decision support of acute stroke treatment.

### BACKGROUND OF THE INVENTION

Therapy of stroke is highly standardized, following international guidelines. A health care value chain analysis revealed that in thrombolytic therapy of stroke there are approximately 1700 different paths, where 40% of treatments follow the basic path, 40% follow a tailored path and 20% contain guideline violations. Hypothesis: most of the guideline violations are not due to ignorance, but instead the result of experts' tacit knowledge - "The art of medicine". The challenge is how to capture this tacit knowledge.

Acute stroke occurs when the blood supply to the brain through blood vessels is disrupted, injuring brain cells and tissues. Brain tissue needs a constant flow of blood to deliver oxygen to the millions of nerve cells that power human thought and function. Stroke symptoms recognition in early phase is crucial as people who get rapid treatment have a better chance of a more complete recovery, even if the initial stroke is relatively severe. If the blood supply to the brain is disrupted, even briefly, large numbers of brain cells can die within minutes, which may cause serious and permanent damage, since the brain cells cannot regenerate. Prompt and correct treatment to limit the damage means normally a faster and more complete recovery.

There are three main forms of acute stroke:
1) Ischaemic stroke: around 80% of people who have an acute stroke are diagnosed with an ischaemic stroke. This happens when a blood vessel in the brain becomes blocked by a blood clot, or atherosclerosis, which develops because of a buildup of fatty substances in blood vessels. These blocks in blood vessel cuts off the oxygen supply to tissue.
2) Transient ischaemic attack: occasionally, the blockage in the blood vessel is only temporary. This causes a transient ischaemic attack, often called a mini-stroke.
3) Haemorrhagic stroke: a blood vessel in the brain bursts, spilling blood into the brain and making it difficult for oxygen and nutrients to reach the nerve cells. The blood flow can also raise the pressure inside the skull, causing further brain injury.

The stroke severity depends on which blood vessels are affected. A blockage in one of the brain main arteries will cause widespread damage and severe loss of function. A blockage in a minor branch of an artery will cause less damage and a full recovery is more likely. Symptoms of an acute stroke develop fast. Acute stroke often gives no warning and stroke symptoms can develop in minutes. The impact of acute stroke can be disabling but the long-term effects depend on how quickly the acute stroke is recognised and treated, which parts of the brain are affected and what type of blood vessel is involved.

The trend in hospitals emergency rooms is to use modern decision support tools to provide rapid care by triage nurses or junior physicians to patients who suffer injuries or develop sudden, serious symptoms of illness or disease. This arrangement shifts more demanding patient treatment to the lower level in nursing hierarchy and effectively guides patients to right nursing path.

The acute stroke treatment options vary depending on the patient's situation, even though the process is essentially simple and straightforward. The physician or nurse must take into account number of issues and make rapid decisions.

Presently the methods to gather medical data are limited and the amount of gathered data is small. Mostly the data is transferred from paper documents to computer and analyses made from the data are delayed, since analysis requires traditional statistical data handling. The information is still collected manually in several places because the amount of electronically accumulated data is limited.

Some decisions support methods for acute stroke analysis has been developed. However, one problem is that the various components of decision support process have been distributed to various stand-alone applications causing lack of unity. This is very problematic in cases as stroke, because the time window is very short to formulate reliable general view of the acute situation. In some systems the decision support is integrated into the patient information system, but not as a process control and there are no interactive decision support algorithms. One example of system mentioned above is Pulsara's system. For example, in USA the forcing algorithms in use are not yet complete and for example the algorithm does not consist "tacit knowledge", which is essential for decision making. Electronic health records (EHR) collect data based on limited and verbal expression, which is laborious. Statistically significant results are obtained with a delay of several months or a year.

### SUMMARY OF THE INVENTION

The aim of the present invention is to eliminate the aforementioned drawbacks and to provide an inexpensive, functionally reliable and as far as possible discreet method for decision support of abnormal state of object.

The method for utilizing data processing and artificial intelligence (AI) technologies in decision support of abnormal state of object according to the invention is characterized by what is disclosed in the characterization part of claim 1 and use according to the invention is characterized by what is disclosed in the characterization part of claims 8 and 9 and the arrangement according to the invention is characterized by what is disclosed in the characterization part of claim 10. Other embodiments of the invention are characterized by what is disclosed in the other claims.

While rule-based decision support tools can be very efficient and accurate in various problems, developments in the field of machine learning in recent decades offer valuable alternatives when building decision support tools, in particular in the health care domain. The traditional task targeted by the use of machine learning models is the prediction of whether a person will have a specific health problem or not, a typical supervised learning task. However, the present invention requires the use of machine learning models for the more refined problem of selecting the optimal treatment for a patient with a specific health condition, for example stroke.

This type of problem can be approached typically by establishing a well-defined similarity measure: by assessing the similarity of a new patient to existing cases in the available database, one can select the optimal treatment as the one that worked best in case of patients with similar symptoms, patient history and other personal characteristics. Association rule mining is one of the most widely used machine learning approaches to tackle this problem: extracting rules from existing cases in an automatic manner and then utilize the rules with conditions describing new patients in the most appropriate way, i.e. high similarity.

A different, potentially more promising approach is to utilize recommendation systems, in particular hybrid systems combining various individual approaches. In a hybrid approach, one can combine:
(i) collaborative filtering; models that derive the optimal treatment by learning patterns in available data and mapping those patterns to the new patient and
(ii) knowledge-based recommendations; combining rules extracted from numerical and textual data, and the tacit knowledge doctors.
The best model to be used in the actual decision support tool can be identified by assessing the performance of various alternatives using different utility measures: consistency and coverage in case of rules, prediction error in case of other models.

The invention also comprises a computer based arrangement for creating decision support instructions of abnormal state of object, which arrangement comprises for example a knowledge base, memory for storing the results, an application with a user interface, a processor for carrying out the instructions of the application, means for interacting and outputting the instructions and information to a user or to a storage file and means for collecting new data from the executed tasks and saving the data to the knowledge base.

The invention, a method and a computer-based arrangement of decision support for abnormal state of object will hereinafter also be called as a shorter term: system.

The system provides a management and monitoring application with a user interface for the decision support for abnormal state of object. The system can be utilized for example in acute stroke treatment process and hereinafter the invention is described more detailed with the example of acute stroke treatment process embodiment.

The features and benefits of the system of this invention are the following:
Main advantages of the system are: for patient faster and more reliable trauma treatment, for treatment unit efficiency and effectiveness improvement and for health care professionals' better traceability and feedback from the work.

Other larger scale advantages realized by the system are new type of data from true process; Instrument to distribute knowledge - from tacit knowledge to structural knowledge; A means to multiply and unify guided treatment processes, spanning hospitals and hospital districts which improves overall quality, efficiency and comparability; Automatic documentation for the staff about decisions and their reasonings.

Fully digital system which comprise clear user interface, necessary control algorithms, a knowledge base and the protocols based on international literature, against which the application is arranged to compare given task parameters of acute stroke treatment to formulate decision support instructions to users.

One advantage is a good adaptiveness. For an inexperienced user the system gives strong control, but for experienced user the system provides light control that leaves room for experience-based, non-standard care solutions. The control may be carried out for example with traffic lights.

With the system, data is transferred directly to the electronic system, partly directly from digital devices and partly by hand. The information that is collected is more extensive and the available data enables monitoring of deviations in real time so that they can be addressed immediately if needed.

The system is collecting extensively new data. There is a wide predefined data repository but the system extents the data collection from the active use of the system. The system for example analyses and stores the cursor movement in user interface and thus the user's behavior and selections are stored in to the knowledge base. The system also sets decision-making questions to the user - as "you deviate from the standards, are you sure you want to continue?". The system is a self-learning system, which collects continuously data from its usage or corresponding system usages in other locations. The system utilizes this extended data to produce more accurate and reliable results and with faster response times in future tasks.

The system also utilizes gamification methods to improve the effectiveness of the important task execution. The gamification elements in user interface are for example continuous instantaneous transparent feedback, running clock, goals and strong graphics.

The system has data transfer means to transfer information and tasks and the ability to delegate work steps. For example, a physician may delegate the thrombolysis treatment with necessary information to a colleague, or delegate a sub-task to a nurse, so that everyone follows the progress of the task from their own device.

The system has telemedicine feature, that can be connected to the application with ER (enhanced reality) using for example of virtual glasses, whereby an expert physician can see the patient through the ER/VR -glasses used by the remote doctor or nurse.

The system is easily customizable to local conditions, such as units of different service levels or protocols of different organizations. The data collection draws a transparent picture to make the data comparable and the system can compare the actual processes.

One advantage is the teaching feature. The system comprises explanations and instructions for sometimes more complex evaluations and decisions to be made. The system can also be used in training, by generating simulated patients and severe simulated situations.

The system user interface has role specific layers for the system users. These layers can easily be modified to support organizations' specific needs.

The system knowledge base has large amount of accurate data, that is expanding after every executed task. This large and expanding knowledge base enables effective artificial intelligence analytics utilization. It also enables real-time adaptation to patient- and situation-specific processes when the actual treatment process is compared to the previous corresponding processes treatment outcomes.

It is obvious to the person skilled in the art that different embodiments and applications of the invention are not limited to the example described above, but that they may be varied within the scope of the claims presented below. Thus, for example, the method can be used for achieving decision support instructions of computer related problems in help-desk or other similar tasks that utilizes large knowledge base.

## Claims

1. A method for utilizing data processing technology in decision support of abnormal state of object, which method comprises a computer-based system, knowledge base, data transfer means and an application with a user interface, **characterized in that** in the method the application is used to utilise artificial intelligence methods and algorithms providing real-time decision support guidance to the user/users while communicating interactively with the user/users through the user interface.

2. Method according to claim 1, **characterized in that** the computer-based system is used to actively collect new data from the executed tasks, save the data to the knowledge base and utilize the new data in future tasks.

3. Method according to claim 1 or 2, **characterized in that** the system is used to utilize experts' tacit knowledge.

4. Method according to claim 1, 2 or 3, **characterized in that** the system is used to utilize gamification elements in the user interface.

5. Method according to any of the claims above, **characterized in that** the system is used to utilize data transfer means to transfer information and delegate work steps, sub-tasks and tasks to other users.

6. Method according to any of the claims above, **characterized in that** system is used to utilize the knowledge base to teach and train the users.

7. Method according to any of the claims above, **characterized in that** the system is used to utilize telemedicine technologies and features to provide clinical health care from a distance.

8. **Use** of a method according to any of claims above for achieving decision support instructions of acute stroke treatment.

9. **Use** of a method according to any of claims 1-6 for achieving decision support instructions of computer related problems in help-desk.

10. Computer-based **arrangement** according to any of methods and/or uses above for achieving the decision support instruction of abnormal state of object, which arrangement comprises a knowledge base, memory for storing the results, an application with a user interface, a processor for carrying out the instructions of the application, means for interacting and outputting the instructions and information to a user or to a storage file and means for collecting new data from the executed tasks and saving the data to the knowledge base.

11. Computer-based arrangement according to claim 10, **characterized in that** the arrangement comprises necessary control algorithms and protocols based on international literature, against which the application is arranged to compare given task parameters of abnormal state of object to formulate decision support instructions to users.

12. Computer-based arrangement according to claim 10 or 11, **characterized in that** the user interface comprises modifiable role specific layers for the system users.
